# EUROPÄISCHE PATENTSCHRIFT

(11) **EP 1 644 028 B1**
(45) Veröffentlichungstag und Bekanntmachung des Hinweises auf die Patenterteilung: **27.05.2009**
(21) Anmeldenummer: 04738079.5
(22) Anmeldetag: 12.07.2004
(51) Int. Cl.: A61K 38/22, C07K 14/575

(54) **SUBSTANZEN MIT BIOLOGISCHER AKTIVITÄT DES VASOAKTIVEN INTESTINALEN PEPTIDS FÜR DIE BEHANDLUNG VON INTERSTITIELLEN LUNGENERKRANKUNGEN**
BIOLOGICALLY ACTIVE SUBSTANCE OF A VASOACTIVE INTESTINAL PEPTIDE FOR TREATING INTERSTITIAL LUNG INFECTIONS
SUBSTANCES A ACTIVITE BIOLOGIQUE DU PEPTIDE INTESTINAL VASOACTIF POUR LE TRAITEMENT D'AFFECTIONS PULMONAIRES INTERSTITIELLES

(30) Priorität: 14.07.2003 CH 122903
(43) Veröffentlichungstag der Anmeldung: 12.04.2006
(73) Patentinhaber: mondoBIOTECH AG, 6371 Stans (CH)
(72) Erfinder: BEVEC, Dorian, CH-6925 Gentilino (CH)
(74) Vertreter: Arth, Hans-Lothar
(86) Internationale Anmeldenummer: PCT/CH2004/000439
(87) Internationale Veröffentlichungsnummer: WO 2005/004899

(56) Entgegenhaltungen:
- EP-A2- 0 835 662
- WO-A-02/43746
- WO-A-96/09064
- WO-A-03/045330
- WO-A-03/046145
- WO-A-03/051388
- WO-A-03/103702
- WO-A2-03/061680
- ALSURO MIYATA ET AL: "ISOLATION OF A NEUROPEPTIDE CORRESPONDING TO THE N-TERMINAL 27 RESIDUES OF THE PITUITARY ADENYLATE CYCLASE ACTIVATING POLYPEPTIDE WITH 38 RESIDUES (PACAP38)" BIOCHEMICAL AND BIOPHYSICAL RESEARCH COMMUNICATIONS, ACADEMIC PRESS INC. ORLANDO, FL, US, Bd. 170, Nr. 2, 31. Juli 1990 (1990-07-31), Seiten 643-648, XP000138019 ISSN: 0006-291X

### SEQUENCE LISTING

<110> Mondobiotech Laboratories Anstalt
<120> Substanzen mit biologischer Aktivität des Vasoaktiven Intestinalen Peptids für die Behandlung von Interstitiellen Lungenerkrankungen
   <130> PMB-0304 INT
<150> CH 01229/03
   <151> 2003-07-14
<160> 14
<170> PatentIn version 3.1
<210> 1
   <211> 28
   <212> PRT
   <213> Homo sapiens
<400> 1
   His Ser Asp Ala Val Phe Thr Asp Asn Tyr Thr Arg Leu Arg Lys Gln
   1               5                   10                  15
   Met Ala Val Lys Lys Tyr Leu Asn Ser Ile Leu Asn
               20                  25
<210> 2
   <211> 38
   <212> PRT
   <213> Homo sapiens
<400> 2
   His Ser Asp Gly Ile Phe Thr Asp Ser Tyr Ser Arg Tyr Arg Lys Gln
   1               5                   10                  15
   Met Ala Val Lys Lys Tyr Leu Ala Ala Val Leu Gly Lys Arg Tyr Lys
               20                  25                  30
   Gln Arg Val Lys Asn Lys
           35
<210> 3
   <211> 27
   <212> PRT
   <213> Homo sapiens
<400> 3
   His Ser Asp Gly Ile Phe Thr Asp Ser Tyr Ser Arg Tyr Arg Lys Gln
   1               5                   10                  15
   Met Ala Val Lys Lys Tyr Leu Ala Ala Val Leu
               20                  25
<210> 4
   <211> 10
   <212> PRT
   <213> Homo sapiens
<400> 4
   Arg Lys Gln Met Ala Val Lys Lys Tyr Leu
   1               5                   10
<210> 5
   <211> 23
   <212> PRT
   <213> Homo sapiens
<220>
   <221> MISC_FEATURE
   <222> (4)..(8)
   <223> X is any naturally occuring amino acid residue
<400> 5
   Phe Thr Asp Xaa Xaa Xaa Xaa Xaa Arg Lys Gln Met Ala Val Lys Lys
   1               5                   10                  15
   Tyr Leu Asn Ser Ile Leu Asn
               20
<210> 6
   <211> 23
   <212> PRT
   <213> Homo sapiens
<400> 6
   Phe Thr Asp Asn Tyr Thr Arg Leu Arg Lys Gln Met Ala Val Lys Lys
   1               5                   10                  15
   Tyr Leu Asn Ser Ile Leu Asn
               20
<210> 7
   <211> 18
   <212> PRT
   <213> Homo sapiens
<400> 7
   Phe Thr Asp Ser Tyr Ser Arg Tyr Arg Lys Gln Met Ala Val Lys Lys
   1               5                   10                  15
   Tyr Leu
<210> 8
   <211> 23
   <212> PRT
   <213> Homo sapiens
<220>
   <221> MISC_FEATURE
   <222> (4)..(5)
   <223> X is any naturally occuring amino acid residue
<220>
   <221> MISC_FEATURE
   <222> (9)..(13)
   <223> X is any naturally occuring amino acid residue
<400> 8
   His Ser Asp Xaa Xaa Phe Thr Asp Xaa Xaa Xaa Xaa Xaa Arg Lys Gln
   1               5                   10                  15
   Met Ala Val Lys Lys Tyr Leu
               20
<210> 9
   <211> 23
   <212> PRT
   <213> Homo sapiens
<400> 9
   His Ser Asp Ala Val Phe Thr Asp Asn Tyr Thr Arg Leu Arg Lys Gln
   1               5                   10                  15
   Met Ala Val Lys Lys Tyr Leu
               20
<210> 10
   <211> 23
   <212> PRT
   <213> Homo sapiens
<400> 10
   His Ser Asp Gly Ile Phe Thr Asp Ser Tyr Ser Arg Tyr Arg Lys Gln
   1               5                   10                  15
   Met Ala Val Lys Lys Tyr Leu
               20
<210> 11
   <211> 28
   <212> PRT
   <213> Homo sapiens
<220>
   <221> MISC_FEATURE
   <222> (4).. (5)
   <223> X is any naturally occuring amino acid residue
<220>
   <221> MISC_FEATURE
   <222> (9)..(13)
   <223> X is any naturally occuring amino acid residue
<220>
   <221> MISC_FEATURE
   <222> (24)..(28)
   <223> X at positions 24 -27 may be any naturally occuring amino acid re sidue; X at position 28 may be H or any naturally occuring amino acid residue
<400> 11
   His Ser Asp Xaa Xaa Phe Thr Asp Xaa Xaa Xaa Xaa Xaa Arg Lys Gln
   1               5                   10                  15
   Met Ala Val Lys Lys Tyr Leu Xaa Xaa Xaa Xaa Xaa
               20                  25
<210> 12
   <211> 38
   <212> PRT
   <213> Homo sapiens
<220>
   <221> MISC_FEATURE
   <222> (4)..(5)
   <223> X is any naturally occuring amino acid residue
<220>
   <221> MISC_FEATURE
   <222> (9)..(13)
   <223> X is any naturally occuring amino acid residue
<220>
   <221> MISC_FEATURE
   <222> (24)..(38)
   <223> X is any naturally occuring amino acid residue
<400> 12
   His Ser Asp Xaa Xaa Phe Thr Asp Xaa Xaa Xaa Xaa Xaa Arg Lys Gln
   1               5                   10                  15
   Met Ala Val Lys Lys Tyr Leu Xaa Xaa Xaa Xaa Xaa Xaa Xaa Xaa Xaa
               20                  25                  30
   Xaa Xaa Xaa Xaa Xaa Xaa
           35
<210> 13
   <211> 3
   <212> PRT
   <213> Homo sapiens
<400> 13
   Phe Thr Asp
   1
<210> 14
   <211> 3
   <212> PRT
   <213> Homo sapiens
<400> 14
   His Ser Asp
   1

## Beschreibung

Die vorliegende Erfindung bezieht sich auf die Verwendung von biologisch und pharmakologisch hochaktiven Peptiden für die Behandlung von Idiopathische Pulmonare Fibrose (IPF), Hypersensitivitätspneumonitis, oder Diffuse Panbronchiolitis eine ist. Die Peptide, die man laut vorliegender Erfindung für die Behandlung der oben aufgeführter Erkrankungen benutzen kann, beinhalten mindestens eine spezifische, hochkonservierte Aminosäureabfolge, die scheinbar eine wichtige Rolle in Verbindung mit den biologischen Ereignissen spielt, welche in den besagten Krankheiten wichtig sind. Es konnte gezeigt werden, das insbesondere das bekannte und natürlich vorkommende Peptid "Vasoaktives Intestinale Peptid (VIP)" und das "Pituitary Adenylate Cyclase-activating Polypeptid (PACAP)", welche diese spezifischen Sequenzen beinhalten, potente Wirkstoffe sind, die erfolgreich für die Behandlung von Idiopathischer Pulmonarer Fibrose und Hypersensitivitätspneumonitis angewendet werden können. Darüber hinaus offenbart die vorliegende Erfindung Zusammensetzungen, die für die Behandlung von interstitiellen Lungenerkrankungen nützlich sind.

### Hintergrund der Erfindung

### Interstitielle Lungenerkrankungen

Interstitielle Erkrankungen sind eine heterogene Gruppe von chronischen Entzündungsreaktionen in der Lunge. Es existieren verschiedene Formen von interstitiellen Lungenerkrankungen, wie beispielsweise die Idiopathische Pulmonare Fibrose (IPF), die Hypersensitivitätspneumonitis, oder die Diffuse Panbronchiolitis. Die im Verlauf der Erkrankungen auftretenden pathophysiologischen Prozesse werden charakterisiert durch eine Kombination aus Gewebsverletzungen und einer übertriebenen Gewebereparatur, welche eine normale Erhaltung des zellulären Wachstums in eine progressive Narbenbildung treibt. Charakteristische Eigenschaften dieser Reaktion beinhalten eine wiederholte Gewebszerstörung und eine intensive proteolytische Aktivität mit einer Veränderung der Zusammensetzung der extrazellulären Matrixkomponenten. Diese Vorgänge führen zu einer Verschiebung der zellulären Immunantwort hin und zu einem stark induziertem Wachstum des Mesenchymalgewebes.

Zytokine, wie der Tumornekrosefaktor alpha, und andere Vermittler des Wachstums wie der "Transforming growth factor beta1 (TGFbeta)" wurden schon lange mit diesen Prozessen in Verbindung gebracht. TGFbeta ist wahrscheinlich der wichtigste Wachstumsvermittler durch seine stark anregende Wirkung auf das Wachstum des Mesenchymalgewebes und aufgrund seiner Fähigkeit, zelluläre Immunvorgänge zu verändern. TGFbeta ist dafür bekannt, in Tiermodellen schwere pulmonare Fibrosen auszulösen, wenn es selber übermässig stark ausgebildet wird. Eine signifikant erhöhte Überproduktion dieses Wachstumsstoffes ist auch in humanen Lungenfibrosen beobachtet. Die immunverändernde Wirkung des TGFbeta ist ebenfalls bekannt. Diese beinhaltet die Inhibition der Produktion des Stoffes Interferon gamma, die Unterdrückung Interferon gamma abhängiger Immunreaktionen, als auch die Induktion immunsuppressiver CD8+ Lymphozyten. In der Tat wurde bereits seit Jahren beobachtet, dass in Patienten mit progressiven Fibrosen eine Veränderung der zellulären Immunität erfolgt, die unabhängig ist von der ursprünglichen Gewebsverletzung an sich. Neuere Untersuchungen haben gezeigt, dass die progressive Narbenbildung in Idiopathischer Lungenfibrose einhergeht mit der Veränderung der Zytokinbalance in Richtung der sogenannten "T-Helfer Typ 2" Reaktion. Diese Reaktion ist charakterisiert durch den Anstieg der sogenannten Th2 Zytokine wie etwa Interleukin 4 (IL-4), IL-10 und IL-13, und durch eine Verminderung bis hin zum völligen Verlust der Produktion von Interferon gamma, den Hauptmediator der sogenannten "T-Helfer Typ 1" Reaktion. Im Gegensatz zu den chronisch entzündlichen Reaktionen in der Fibrose, ist eine akute Entzündung des Lungeninterstitiums, beispielsweise als Folge einer Infektion mit Bakterien, durch eine gleichzeitige Produktion der T-Helfer Typ 1 und T-Helfer Typ 2 Zytokine, wie beispielsweise des Interferons gamma, IL-12 und IL-4 gekennzeichnet. Zusätzlich dazu erfolgt auch eine gesteigerte Produktion des TGFbeta, wahrscheinlich als Zeichen der aktivierten Wundheilung.

Aufgrund der üblicherweise erst späten Erkennung der Idiopathischen Lungenfibrose sind die frühen zellulären Vorgänge in der Erkrankung beinahe nicht erkennbar. Die Wundheilungsintensität wird direkt beeinflusst durch Entzündungsmechanismen, welche ihrerseits einen erhöhten Umsatz an extrazellulären Matrixkomponenten und anderer zellulären Komponenten verursachen. Chronische Entzündungen, üblicherweise durch Infektionen verursacht, führen zu pathologisch übersteigerten Wundheilungsreaktionen. Selbst im Falle das man die entzündungsverursachenden Agentien kennt, gibt es momentan keine Medikamente auf dem Markt, mit deren Hilfe man Organfibrosen erfolgreich behandeln kann. Millionen Leute sterben durch eine langsame Zerstörung der lebensnotwendigen Organsysteme durch eine pathologische Umstrukturierung ihrer Funktionsgewebe. Dieser Prozess wird als Fibrose beschrieben der durch fibropreliferative Mechanismen ausgelöst und gesteuert. Die einzige Lösung heutzutage sind Organtransplantationen, die mit vielen Risiken und intensiven Kosten verbunden sind.

Die fibroproliferativen Reaktionen betreffen alle Organe des Körpers. In dem Gasaustaschgewebe der Lunge kennt man es als Lungenfibrose, in der Leber als Zirrhose, in der Niere als Glomerulosclerosis. Diese Zustände werden kollektiv als fibroproliferative Erkrankungen bezeichnet. In der Fibrose wird das gesunde Gewebe progressiv durch Bindegewebe und

Unterstützungsgewebe ersetzt. Dieser Prozess basiert auf pathologisch beschleunigtem Wachstum der Gewebezellen, welche üblicherweise für die Durchführung der normalen Wundheilung zuständig sind. Deshalb definiert man die fibroproliferativen Erkrankungen als Erkrankungen der unkontralliert beschleunigten Wundheilung. In der Fibrose wird das funktionelle Organgewebe bis zum völligen Organfunktionsverlust ersetzt.

Heutzutage kennt man mehr als 150 verschiedene Mechanismen, durch deren Einwirkung es zu einer Beschädigung der Lunge kommen kann, was an sich wiederum eine fibroproliferative Wundheilung auslösen kann.

Zu diesen krankheitsauslösenden Mechanismen gehören chronische Infektionen, Staubexpositionen organischen oder anorganischen Ursprungs, verschiedene Medikamente und

Autoimmunmechanismen. Trotzdem ist der fibrotische Prozess an sich ein eigenständiger Vorgang. Kleinere Entzündungen können zu einer dramatischen Beschleunigung des Fibrosierungsprozesses führen. Chronische virale Erkrankungen sind wahrscheinlich die häufigsten Ursachen für progressive Fibrosen, trotz immunosuppressiver Behandlungen. Ähnliches gilt auch für chronische bakterielle und fungale Infektionen, mit oder ohne gastrische Aufstossreaktionen, welche chronische Entzündungen der terminalen Bronchien auslösen können, die einen unmittelbaren Einfluss auf den chronischen Wundheilungsprozess in den benachbarten Alveolen haben. Auf zellulärer Ebene spielen die antigenpräsentierenden Zellen, wie es die dendritischen Zellen welche sind, eine wichtige Rolle in der Pathophysiologie der erwähnten Erkrankungen. Durch ihre Überfunktion aktivieren sie zu stark etwa die T-Lymphozyten, was zu einer chronischen Immunaktivierung führen kann. Deshalb sind Substanzen für die Behandlung obengenannter Erkrankungen wünschenswert, die eine Inhibition der überschiessenden Aktivität der dendritischen Zellen herbeiführen können.

### Zusammenfassung der Erfindung

Die Erfindung beschreibt erstmalig die präklinisch/zelluläre und klinische Relevanz von VIP, PACAP und Substanzen mit der biologischen Aktivität von VIP oder PACAP für die Behandlung von Idiopathischen Pulmonaren Fibrose, Hypersensitivitätspneumonitis und Diffusen Panbronchiolitis.

VIP und PACAP werden in verschiedenen Arealen des zentralen Nervensystems synthetisiert, zum Beispiel in spezifischen Gehirnregionen wie dem Hippocampus und Cortex, als auch in peripheren Ganglien. VIP wird des weiteren von Immunzellen freigesetzt.

### Vasoaktives Intestinales Peptid (VIP):

VIP ist ein Peptid das aus 28 Aminosäuren besteht und folgende Aminosäureabfolge aufweist (vom N- zum C-terminus) :

Gesunde Personen weisen VIP Konzentrationen von etwa 40 pg/ml Serum auf.

VIP ist ein weitverbreitetes Neurohormon, das eine grosse Anzahl physiologischer Effekte steuert, wie etwa die gastrointestinale Sekretion, die Entspannung der gastrointestinalen glatten Muskulatur, oder die Lipolyse in Adipozyten. Unter physiologischen Bedingungen ist VIP ein neuroendokriner Mediator. Berichte in der Literatur weisen auf die regulatorische Wirkung von VIP bezüglich Wachstum und Proliferation sowohl in gesunden, als auch in malignen Zellen hin(Hultgardh et al. Growth-inhibitory properties of vasoactive intestinal polypeptide. Regul. Pept. 22, 267-274. 1988). Die biologischen Effekte werden durch spezifische Rezeptoren vermittelt (VIP-R), die auf den Oberflächen verschiedener Zelltypen lokalisiert sind(Ishiharaet al., Functional expression and tissue distribution of a novel receptor for vasoactive intestinal polypeptide. Neuron 8, 811-819. 1992). VIP beeinflusst das Wachstum maligner Zellen aus Neuroblastomen, Brust-, Lungen- und Darmkrebs (Moody et al., Proc. Natl. Acad. Sci. USA, 90, 4345, 1993), indem es die eigenen Rezeptoren induziert. In einigen Fällen induziert das VIP dosisabhängig Zellteilung (Wollman et al., Brain Res., 624, 339, 1993). VIP und biologisch funktionelle Analoge und Derivate weisen entspannende Wirkung auf glatte Muskulatur auf(Maruno et al., VIP inhibits basal and histamine-stimulated proliferation of human airway smooth muscle cells. Am.J.Physiol. 268, L1047-L1051, 1995), zeigen bronchodilatorische Aktivität ohne starke cardiovaskuläre Nebeneffekte, haben Effekte in Asthma, im Bluthochdruck, Impotenz, Ischaemie und in neurologischen Erkrankungen wie der Alzheimer Erkrankung (z.B. WO 9106565*,* EP 0536741*,* US 3,880,826*,* EP 0204447*,* EP 0405242*,* WO 9527496*,* EP 0463450*,* EP 0613904*,* EP 0663406*,* WO 9735561*,* EP 0620008).

VIP Rezeptoren fand man im Epithel der Trachea und der Bronchiolen. Sie sind ebenfalls auf Makrophagen, die Kapillaren umgeben exprimiert, im Bindegewebe der Trachea und Bronchien, in alveolären Wänden und in der Auskleidung der Lungenvenen und Lungenarterien. Pepiderge Nervenfasern scheinen in der Lunge für die Synthese von VIP zuständig zu sein (Dey et al., Localization of VIP-immunoreactive nerves in airways and pulmonary vessels of dogs, cat, and human subjects. Cell and Tissue Research 220, 231-238. 1981; Said, S. I. Vasoactive intestinal polypeptide (VIP) in asthma. Ann.N.Y.Acad.Sci. 629, 305-318. 1991). VIP reduziert den Widerstand im Lungengewebe (Hamasaki et al., Relaxant action of VIP on cat pulmonary artery: comparison with acetylcholine, isoproterenol, and PGE1. J.Appl.Physiol. 54, 1607-1611. 1983; Iwabuchi et al., Vasoactive intestinal peptide causes nitric oxide-dependent pulmonary vasodilation in isolated rat lung. Respiration 64, 54-58. 1997; Saga, T. and Said, S. I. Vasoactive intestinal peptide relaxes isolated strips of human bronchus, pulmonary artery, and lung parenchyma. Trans.Assoc.Am.Physicians. 97, 304-310. 1984). Weitere Studien zeigen eine hohe Expressionsrate von VIP-R in der Lunge, gekennzeichnet durch eine hohe Aufnahmerate radioaktiv markierten VIPs (Radereret al., 123I-labelled vasoactive intestinal peptide receptor scintigraphy in patients with colorectal cancer. Br.J.Cancer 78, 1-5. 1998; Raderer et al., Iodine-123-vasoactive intestinal peptide receptor scanning in patients with pancreatic cancer. J.Nucl.Med. 39, 1570-1575. 1998; Raderer et al., Value of peptide receptor scintigraphy using (123)I-vasoactive intestinal peptide and (111)In-DTPA-D-Phel-octreotide in 194 carcinoid patients: Vienna University Experience, 1993 to 1998. J.Clin.Oncol. 18, 1331-1336. 2000; Virgolini et al., Vasoactive intestinal peptide receptor scintigraphy. J.Nucl.Med. 36, 1732-1739. 1995).

### Pituitary adenylate cyclase-activating polypeptide (PACAP):

PACAP ist ein Neuropeptid bestehend aus 38 Aminosäuren mit folgender Aminosäureabfolge (vom N- zum C-terminus):

Es wurden zwei Peptidformen identifiziert: PACAP-38 und das C-terminal verkürzte PACAP-27. PACAP-27, welches 68% Homologie zum VIP aufweist, hat die folgende Aminosäureabfolge (vom N- zum C-terminus):

PACAP ist ein starker Stimulator der Adenylatcyclase und induziert somit das zyklische adenosine 3, 5 -monophosphat (cAMP) in verschiedenen Zellen. Der Wirkstoff agiert als ein Hormon des Hypothalamus als Neurotransmitter, Neuromodulator, Vasodilator und neurotropher Factor. PACAP stimuliert auch die Ausschüttung von Insulin. PACAP ist als neurotropher Faktor an der Entwicklung des Gehirns während der Embryogenese beteiligt. Im entwickelten Gehirn scheint PACAP als ein neuroprotektiver Faktor zu wirken, der eine neuronale Zerstörung durch multiple Verletzungen verhindert. PACAP ist weit verbreitet im Gehirn und peripheren Organen, insbesondere in der Bauchspeicheldrüse, den Gonaden und im respiratorischen Trakt. Drei PACAP Rezeptoren sind beschrieben. Der Typ I Rezeptor weist eine hohe Affinität für PACAP (und eine sehr niedrige Affinität für VIP) auf, während der Typ II Rezeptor ähnliche Affinitäten für PACAP und VIP aufweist. Es gibt einen weiteren, PACAP-spezifischen Rezeptor PAC1.

In der vorliegenden Erfindung beschreiben wir den Gebrauch von bekannten Wirkstoffen, die zur Herstellung eines Medikamentes für die Vorbeugung und/oder Behandlung von Idiopathischen Lungenfibrose, Hypersensitivitätspneumonitis, oder Diffusen Panbronchiolitis geeignet sind.

Überraschenderweise fanden wir, dass Peptide mit der hochkonservierten Dekapeptidaminosäureabfolge Arg-Lys-Gln-Met-Ala-Val-Lys-Lys-Tyr-Leu (SEQ ID No. 4) hocheffektive Hemmer der Maturierung dendritischer Zellen sind, und hocheffektiv sind wenn sie Patienten verabreicht werden, welche an Idiopathischer Lungenfibrose, an Hypersensitivitätspneumonitis, oder an Diffuser Panbronchiolitis leiden. Substanzen werden bevorzugt, welche diese Aminosäureabfolge beinhalten und insgesamt 10 - 60, vorzugsweise 10 - 38, am meisten bevorzugt 10 - 28 oder 10 - 27 Aminosäuren beinhalten und die identischen biologischen Eigenschaften wie das VIP oder PACAP aufweisen, die ebenfalls die entsprechende konservierte Aminosäureabfolge beinhalten.

Im allgemeinen wurde gefunden, dass VIP- und PACAP-ähnliche Peptide und Polypeptide, welche die obenerwähnte therapeutische Funktion und Effizienz ausüben, die folgende Aminosäureabfolge überstreichen:
(A)ₙ-Arg-Lys-Gln-Met-Ala-Val-Lys-Lys-Tyr-Leu-(B)ₘ
wobei A, B jede natürliche Aminosäure darstellt und A und B voneinander unabhängig sind; n, m sind Platzhalter mit Werten von 0 - 25; n und m sind voneinander unabhängig. Der Wert von m ist vorzugsweise 4 - 18, mehr bevorzugt 5 - 15, am meisten bevorzugt 10 - 15.

Polypeptide oder Peptide, wobei (A)ₙ (falls n > 2) die Tripeptidsequenz His-Ser-Asp (SEQ ID No. 14) und/oder Phe-Thr-Asp (SEQ ID No. 13) in N-terminaler Richtung die oben spezifizierte Dekapeptidsequenz überstreichen (1 - 10 Aminosäurenstellen) besitzen eine erhöhte Aktivität.

Speziell verbesserte Aktivität zeigen Polypeptide, wobei (A)ₙ (falls n > 2) die Bedeutung von (X)ₒ-Phe-Thr-Asp-(Y)ₚ und (X) ₒ (falls o > 2) die Bedeutung von (X')_{q}-His-Ser-Asp-(X'')ᵣ hat wobei X, Y, X', X'' eine natürliche Aminosäure darstellt; und o, p, ist ein Platzhalter für die Werte von 0 - 11, und r, q sind Platzhalter mit Werten von 0 - 4. Bevorzugt werden die Werte von o und p von 0 - 8, mehr bevorzugt die Werte von 1 - 5. Bevorzugte Werte von r sind 0 - 2.

Bevorzugte Beispiele, die unter die beschriebene generische Formel fallen, haben folgende Aminosäurenabfolgen

Zusammenfassend kann man sagen, dass die vorliegende Erfindung folgenden Gegenstand beschreibt:

Den Gebrauch einer Substanz für die Herstellung eines Medikamentes zur Behandlung von Idiopathischen Lungenfibrose oder Hypersensitivitätspneumonitis und deren Anwendung an Patienten, wobei die Substanz die biologische Aktivität von VIP oder PACAP aufweist.

### Detaillierte Beschreibung

Geeignete Substanzen, welche die therapeutischen Effekte gemäss der Erfindung aufweisen, sind jene, welche die gleiche, aber auch verminderte oder erhöhte biologische Aktivität des VIP oder PACAP aufweisen. Bevorzugt sind die

Die therapeutische Verwendung von VIP und verwandten Peptiden ist bereits bekannt. Beispielsweise offenbart die internationale Offenlegungsschrift WO 03/046145 A die Verwendung von VIP bei der Behandlung von Hypertonie und der gezielten Therapie von Brustkrebszellen, WO 03/045330 A offenbart die Verwendung von VIP bei der Behandlung von Hypertonie und collageninduzierter Arthritis und der gezielten Therapie von Brustkrebszellen, WO 02/043746 A offenbart die Verwendung von VIP bei der Behandlung von Hypertonie, WO 03/061680 A offenbart die Verwendung eines Peptids umfassend SEQ ID NO: 4 und SEQ ID NO: 13 oder SEQ ID NO: 4, SEQ ID NO: 13 und SEQ ID NO: 14, wie in der vorliegenden Anmeldung beschrieben, für die Behandlung von Erkrankungen in Verbindung mit COPD (chronic obstructive pulmonary disease), einschließlich chronische Bronchitis, Bronchiektasie und Emphysem und für die Behandlung von ARDS (acute adult respiratory distress syndrome) und WO 03/103702 A offenbart die Verwendung eines Peptids umfassend SEQ ID NO: 4 und SEQ ID NO: 13 oder SEQ ID NO: 4, SEQ ID NO: 13 und SEQ ID NO: 14, wie in der vorliegenden Anmeldung beschrieben, für die Behandlung von Sarkoidose und sekundäre Lungenhypertonie. Behandlungen für die interstitielle Lungenerkrankung der idiopathischen Lungenfibrose sind ebenfalls bekannt, beispielsweise offenbart WO 03/051388 A die Verwendung von Interferongamma für die Behandlung der idiopathischen Lungenfibrose. Jedoch wurde die Verwendung von VIP und verwandten Peptiden für die Behandlung von idiopathischer Lungenfibrose, hypersensitiver Pneumonie oder diffuser Panbronchiolitis, wie in der vorliegenden Anmeldung beansprucht, zuvor nicht offenbart.

Substanzen gemäss der Erfindung, welche die gleiche oder erhöhte biologische Aktivität aufweisen. Alle Substanzen die in diese Gruppe fallen, überstreichen die Aminosäureabfolge Arg-Lys-Gln-Met-Ala-Val-Lys-Lys-Tyr-Leu (SEQ ID No. 4).

Die Erfindung beinhaltet ebenfalls Derivate der offenbarten Peptide und Polypeptide mit gleicher biologischen Aktivität.

Der Ausdruck **"gleiche biologische Aktivität"** bedeutet die biologische, physiologische oder therapeutische Aktivität oder Funktionalität verglichen mit den relevanen Eigenschaften der genannten Peptide und Polypeptide, bevorzugt mit denen von VIP oder PACAP.

Der Ausdruck **"Derivat"** bedeutet eine Peptidsubstanz, welche mehr oder weniger direkt vom korrespondierenden Peptid herstammt, wie etwa von VIP oder PACAP und welche durch einige Additionen, Deletionen, Mutationen oder Modifikationen verändert werden, ohne die biologischen Eigenschaften der Ursprungspeptide zu ändern. Verwendbare VIP Derivate sind beispielsweise WO 8905857, WO 9106565, EP 0663406 und WO 9729126 (Fmoc geschütztes VIP) offenbart. Der Ausdruck beinhaltet auch Konjugate der Peptide und Polypeptide gemäss der Erfindung, welche aus dem Ursprungspeptid oder Polypeptid bestehen und an lipophile Stoffe wie etwa Liposomen gekoppelt sind. VIP - Liposomen sind beispielsweise in WO 9527496 oder WO 9735561 offenbart und besitzen verbesserte Eigenschaften hinsichtlich Bioverfügbarkeit und Schutz gegen proteolytische Spaltung. Darüber hinaus beinhaltet der Ausdruck auch Fragmente und leicht veränderte Fragmente wie verkürzter Formen.

Der Ausdruck **"Analog"** bedeutet eine Substanz, welche eine andere Struktur und Zusammensetzung verglichen mit den Polypeptiden und Peptiden der Erfindung aufweisen, bevorzugt mit der von VIP, jedoch ohne Veränderung der biologischen Eigenschaften. VIP Analoge können natürliche oder synthetische Peptide, aber auch Nichtpeptide sein. Gemäss der Erfindung sind VIP Analoge Peptide. Beispiele für offenbarte VIP Analoge findet man in EP 0325044 (zyklische Peptide), EP 0225020 (lineare Peptide), EP 0536741 (zyklische VIP Modifikationen), EP 0405242, EP 0184309 and EP 0613904. Der Ausdruck beinhaltet auch VIP oder PACAP Homologe, die nicht VIP oder PACAP sind, aber grosse Strukturähnlichkeiten zu VIP aufzeigen. Solch ein VIP Homolog ist gemäss der Erfindung das PACAP selbst und dessen verkürzte Form PACAP-27. Bevorzugte VIP/PACAP Homologe sind Peptide, welche eine oder mehrere Konsensusaminosäureabfolgen überstreichen. Beispiele hierfür sind das Peptid histidin isoleucin (PHI), Peptid histidin methionin (PHM), das Peptide "human growth hormone releasing factor" (GRF), PACAP, Secretin und Glucagon.

Der Ausdruck **"stabilisierte Form"** beschreibt ein Derivat oder Analog, wobei das Ursprungspeptid verändert wurde, um grössere Stabilität und eine erhöhte Halbwertszeit im Blut und Serum zu gewinnen. Solche stabilisierte Formen werden bevorzugt, wenn das Polypeptid durch Enzymaktivität fragmentiert wird. Mögliche stabilisierte Formen sind zyklische Peptide oder Polypeptide, wie etwa das zyklische VIP oder das zyklische PACAP, Fusionsproteine, bevorzugt Fc-Fusionsproteine oder pegyliert Polypeptide, beispielsweise pegyliertes VIP oder PACAP. Methoden zur Herstellung solcher Polypeptide sind im Stand der Technik bekannt. Polypeptide und Proteine können gegen Proteolyse durch Anheftung chemischer Gruppen geschützt werden. Solche Anheftungen können effektiv verhindern, dass die proteolytischen Enzyme physischen Kontakt mit der Proteinstruktur eingehen und so die Degradation verhindern. Polyethylenglycol ist eine solcher chemischen Strukturen, die nachweislich gegen Proteolyse schützt (Sada, et al., J. Fermentation Bioengineering 71: 137-139, 1991). Zusätzlich zum Schutz gegen proteolytische Spaltungen ist bekannt, dass chemische Modifikationen biologisch aktiver Proteine unter Umständen weitere Vorteile bieten, wie etwa die Erhöhung der Stabilität und Verweildauer in der Zirkulation, oder eine Verminderung der Immunogenität. (US. 4,179,337; Abuchowski et al., Enzymes as Drugs.; J.S. Holcerberg and J. Roberts, eds. pp. 367-383, 1981; Francis, Focus on Growth Factors 3: 4-10; EP 0 401 384).

Der Ausdruck **"Fusionsprotein"** bedeutet dass eine Substanz, speziell in der stabilisierten Form aus einem Polypeptid gemäss der Erfindung, bevorzugt VIP oder einem VIP Derivat oder Analog, so wie PACAP, besteht, mit einem anderen Peptid oder Protein fusioniert wird. Solch ein Protein ist bevorzugt ein Immunglobulinmolekül, mehr bevorzugt ein Fragment davon, am meisten bevorzugt eine Fc-Portion eines IgG Moleküls, bevorzugt ein IgG1. Ein Fc-VIP-Fusionsprotein ist offenbart in WO 200024278 und weist eine verbesserte Halbwerteszeit im Serum und Blut auf. Ein weiteres Beispiel ist das Fc-PACAP und das FC-PACAP-27.

Die Substanz gemäss der Erfindung kann zur Herstellung eines Medikamentes oder eines Diagnostikums verwendet werden, um die pathologischen Eigenschaften in einem Individuum zu evaluieren.

Der Ausdruck **"Individuum"** bezieht sich bevorzugt auf Säuger, insbesondere auf Menschen. Die Substanz wird in pharmazeutischen Zusammensetzungen und Formulierungen benutzt, in welchen als eine Regel, pharmazeutisch akzeptable Trägermaterialien oder Lösungsmittel eingesetzt werden. Techniken für die Formulierung und Anwendung der Substanzen aus der vorliegenden Erfindung kann man den "Remington's Pharmaceutical Sciences" Mack Publishing Co., Easton PA, entnehmen.

Wie hier verwendet, bedeutet der Ausdruck **"pharmazeutisch akzeptabler Träger"** einen inerten, nicht toxischen festen oder flüssigen Füllstoff.

Für Inhalationen ist die Substanz gemäss der Erfindung bevorzugt in aerosolische Form zu bringen. Aerosole und Techniken zu deren Herstellung sind Im Stand der Technik bekannt. Aerosole, die man mittels Inhalationsapparaten appliziert und die ein Peptid oder Polypeptid der vorliegenden Erfindung beinhalten, beispielsweise VIP oder PACAP sind bevorzugt, falls direkt pulmonale Symptome behandelt werden müssen.

Therapeutische Einheiten der Substanz gemäss der Erfindung können täglich auf einmal, oder durch Mehrfachgabe kleinerer Dosen appliziert werden.

### Kombinationstherapie

Die Substanzen der Erfindung können einem Patienten entweder als Einzelsubstanz oder in Kombination mit anderen Wirkstoffen verabreicht werden, wie etwa mit Calcium-Kanalblockern (diltiazem), Immunosppressiven Substanzen (prednisolone), Antimikrobiellen Agentien wie Antibiotika oder Bakteriophagen, die spezifisch gegen entweder Staphylokokken, Pseudomonaden, Burkholderia, Haemophilus, Streptokokken, oder andere Bakterien in der Lunge wirken, Beta-adrenergische Rezeptor-blockierende Substanzen und Angiotensin-rezeptor-antagonisten oder Angiotensinconverting-enzyme-inhibitors (ramipril), Antiproliferative Substanzen (atorvastatin), Endothelin-rezeptor-antagonisten, (Bosentan, Altrasentan, Sitaxsentan, Enrasentan, BMS 193884, Darusentan, TBC 3711, BSF 208075, BSF 302146, SPP 301), oder anderen antiproliferativen Substanzen (D-24851, Imatinib mesylate). Die vorliegende Erfindung bezieht sich auch auf die Kombinationstherapie der offenbarten Peptide mit zumindest einem der obenerwähnten Medikamente. -

Überraschenderweise fanden wir, dass die Peptide und Polypeptide, wie oben und in den Patentansprüchen definiert, insbesondere VIP und PACAP, Inhibitoren der Reifung von humanen dendritischen Zellen sind und positive Effekte in der Behandlung von Patienten mit Idiopathischer Lungenfibrose, Hypersensitivitätspneumonitis und Diffuser Panbronchiolitis aufweisen.

### Beispiel

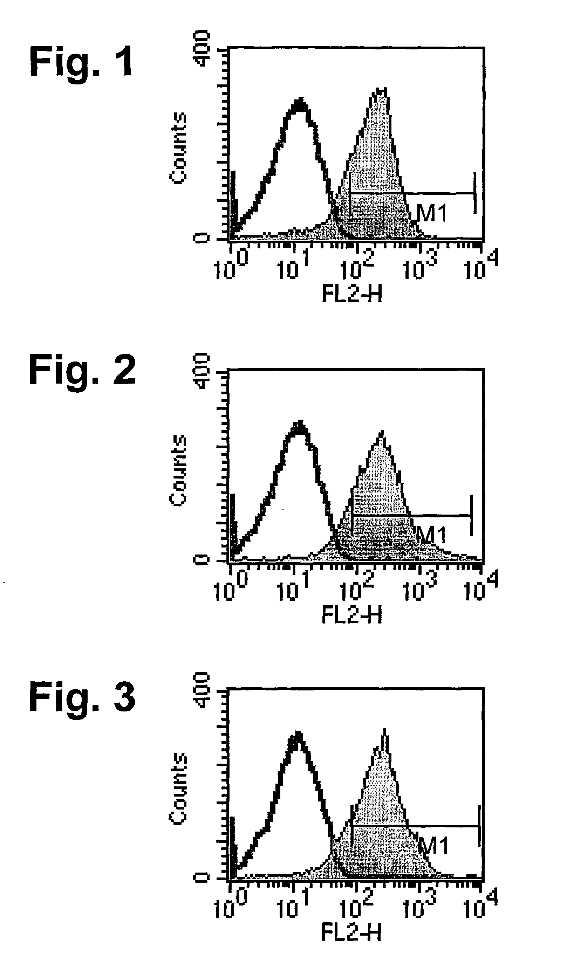

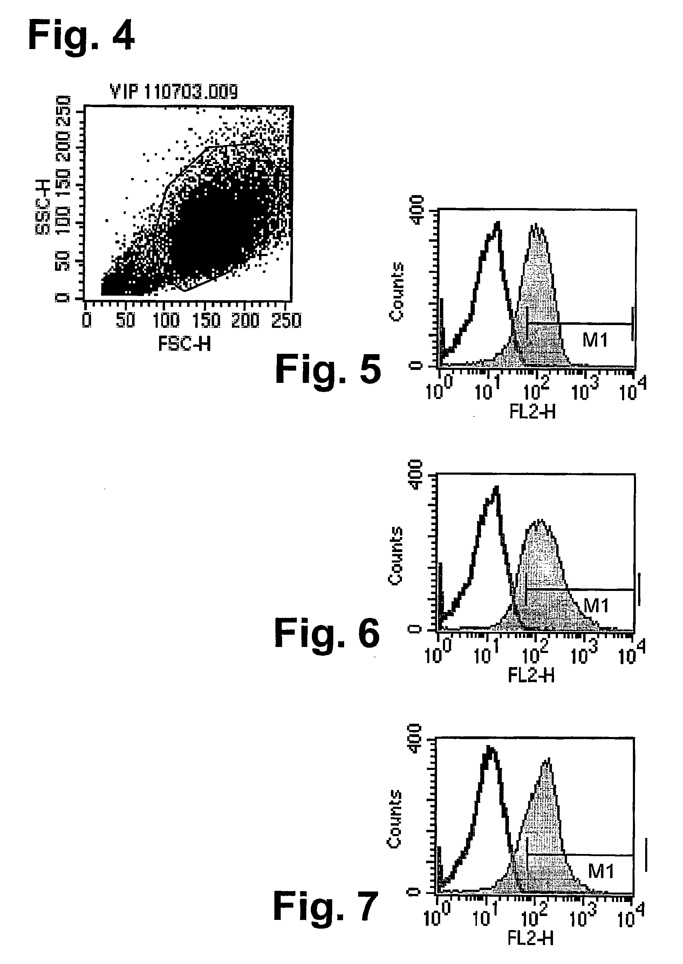

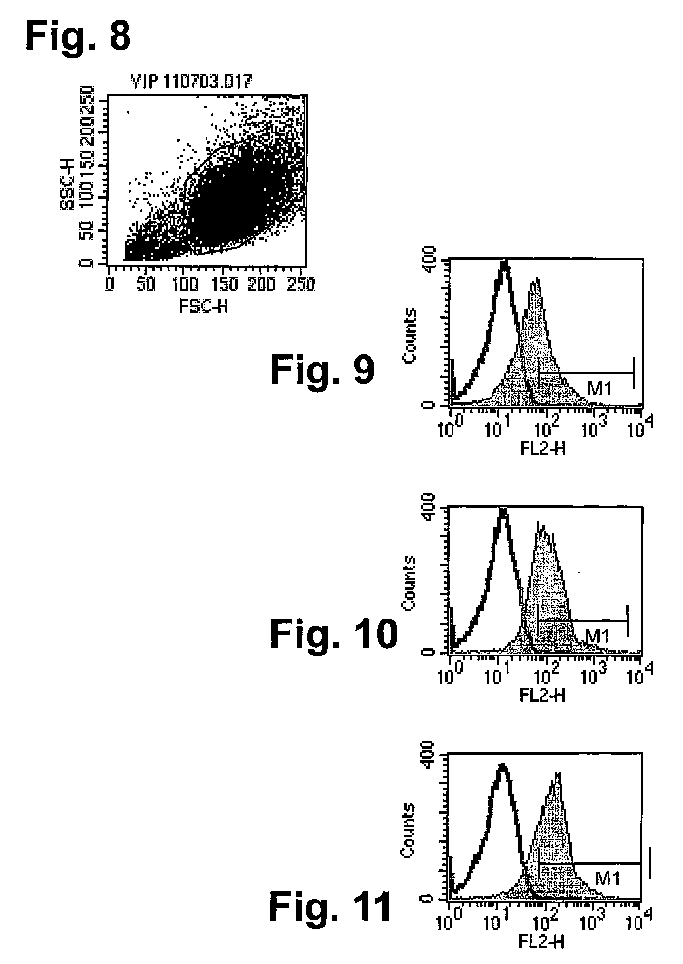

Gezeigt wird in den Figuren 1-11 der Effekt von VIP auf die Reifung von humanen dendritischen Zellen. CD 83 ist ein charakteristisches Oberflächenprotein auf ausgereiften dendritischen Zellen. Wenn sein Vorkommen auf den Zellen gering ist, so heisst das, dass die Zellen noch unreif sind und ihre biologische Funktion nicht ausüben können. Bei Anwendung von 1 micromol VIP (Fig. 5), bzw. besonders bei 9.1 micromol VIP (Fig. 9) zeigt sich ein dramatischer Rückgang der CD 83 Oberflächenmoleküle. Dieser Rückgang wird demonstriert durch den Wert M1 38.22 - bei der Messung mit VIP 9.1 micromol - verglichen mit dem Wert M1 81.26 (Fig. 10) - bei der Messung unbehandelter Zellen (Fig. 1) - in der sogenannten fluoreszenzaktivierten Zellanalyse, bei welcher mit fluoreszenzmarkierten Antikörpern die Zelloberflächen nach den entsprechenden Antigenen (in diesem Fall CD83) abgesucht werden. Der Effekt von VIP auf das CD 83 Molekül ist spezifisch, da andere Oberflächenmoleküle - wie das sogenannte MHC I (siehe Figuren 2, 6 und 10) oder MHC II (siehe Figuren 3, 7 und 11) - bei gleicher VIP Behandlung sehr viel weniger unterdrückt werden. Dies zeigt sich im Wert M1 70.57 für MHC I bei 9.1 micromol VIP im Vergleich zum Wert M1 85.59 für MHC I bei unbehandelten Zellen sowie im Wert M1 77.73 für MHC II bei 9.1 micromol VIP im Vergleich zum Wert M1 83.94 für MHC II bei unbehandelten Zellen.

### Tabellen:

### Unbehandelte Zellen:

CD 83 Fig. 1

| Marker | % Gated | Mean |
|---|---|---|
| All | 100.00 | 202.43 |
| M1 | 81.26 | 238.31 |

MHC I Fig. 2

| Marker | % Gated | Mean |
|---|---|---|
| All | 100.00 | 352.27 |
| M1 | 85.59 | 398.25 |

MHC II Fig. 3

| Marker | % Gated | Mean |
|---|---|---|
| All | 100.00 | 290.14 |
| M1 | 83.94 | 335.90 |

### VIP (1 µM):

CD 83 Fig. 6

| Marker | % Gated | Mean |
|---|---|---|
| All | 100.00 | 108.13 |
| M1 | 69.70 | 137.30 |

MHC I Fig. 7

| Marker | % Gated | Mean |
|---|---|---|
| All | 100.00 | 200.61 |
| M1 | 78.69 | 242.97 |

MHC II Fig. 8

| Marker | % Gated | Mean |
|---|---|---|
| All | 100.00 | 170.33 |
| M1 | 77.41 | 207.21 |

### VIP (9.1 µM):

CD 83 Fig. 10

| Marker | % Gated | Mean |
|---|---|---|
| All | 100.00 | 76.48 |
| M1 | 38.22 | 143.06 |

MHC I Fig. 11

| Marker | % Gated | Mean |
|---|---|---|
| All | 100.00 | 136.67 |
| M1 | 70.57 | 174.61 |

MHC II Fig. 12

| Marker | % Gated | Mean |
|---|---|---|
| All | 100.00 | 175.73 |
| M1 | 77.73 | 213.25 |

### Verwendete Abkürzungen:

VIP = Vasoaktives Intestinale Peptid
PACAP = Pituitary Adenylate Cyclase-activating Polypeptid
IPF = Idiopathische Pulmonare Fibrose
TGFbeta = Transforming growth factor beta1
cAMP = Zyklisches Adenosine 3, 5 -monophosphat
PHI = Peptid histidin isoleucin
PHM = Peptid histidin methionin
GRF = Peptide "human growth hormone releasing factor"
CD 83 = Cluster of Differentiation Nummer 83
MHC I = Major Histocompatibility Complex class 1 antigen
MHC II = Major Histocompatibility Complex class 2 antigen

## Patentansprüche

1. Verwendung einer Substanz für die Herstellung eines Medikamentes für die Behandlung von Patienten, die an Idiopathischer Pulmonarer Fibrose, Hypersensitivitätspneumonitis oder Diffuser Panbronchiolitis leiden, wobei die besagte Substanz ein Peptid oder ein Polypeptid ist, welches die folgende Aminosäurenabfolge beinhaltet:
Arg-Lys-Gln-Met-Ala-Val-Lys-Lys-Tyr-Leu (SEQ ID Nr. 4).

2. Verwendung gemäß Anspruch 1, wobei das besagte Peptid oder ein Polypeptid des Weiteren eine Aminosäureabfolge folgender Sequenz überstreicht:
His-Ser-Asp (SEQ ID Nr. 14); Phe-Thr-Asp (SEQ ID Nr. 13).

3. Verwendung gemäß Anspruch 1, wobei das besagte Peptid oder ein Polypeptid folgende Aminosäureabfolge aufweist:
(A)ₙ-Arg-Lys-Gln-Met-Ala-Val-Lys-Lys-Tyr-Leu-(B)ₘ;
wobei A und B jeweils unabhängig voneinander jede natürliche vorkommende Aminosäure darstellen können; und n und m jeweils unabhängig voneinander ein Platzhalter für Werte von 0 - 25 sein können.

4. Verwendung gemäß Anspruch 3, wobei, falls n > 2, (A)ₙ folgende Sequenz aufweist:
(X)ₒ-Phe-Thr-Asp-(Y)ₚ;
wobei X und Y jeweils unabhängig voneinander jede natürliche vorkommende Aminosäure darstellen können; und o und p jeweils unabhängig voneinander ein Platzhalter für Werte von 0 - 11 sein können.

5. Verwendung gemäß Anspruch 4, wobei, falls o > 2, (X)ₒ folgende Sequenz aufweist:
(X')_{q}-His-Ser-Asp-(X")ᵣ;
wobei X' und X" jeweils unabhängig voneinander jede natürliche vorkommende Aminosäure darstellen können; und r und q jeweils unabhängig voneinander ein Platzhalter für Werte von 0 - 4 sein können.

6. Verwendung gemäß Anspruch 3, wobei die Sequenz des beschriebenen Peptids oder Polypeptids aus folgender Gruppe ausgewählt wird:
(i) Arg-Lys-Gln-Met-Ala-Val-Lys-Lys-Tyr-Leu (SEQ ID Nr. 4)
(ii)
(iii)
(iv)
(v)
(vi)
(vii)
(viii)
(ix)
(x)
(xi)
wobei X¹ - X²² jede natürlich vorkommende Aminosäure sein kann.

7. Verwendung gemäß der Ansprüche 1-6, wobei jedes der genannten Peptide oder Polypeptide in einer stabilisierten Form vorliegt.

8. Verwendung gemäß der Ansprüche 1 - 7, wobei die genannte Erkrankung Idiopathische Pulmonare Fibrose ist.

9. Verwendung gemäß der Ansprüche 1 - 7, wobei die genannte Erkrankung Hypersensitivitätspneumonitis ist.

10. Verwendung gemäß der Ansprüche 1 - 7, wobei die genannte Erkrankung Diffuse Panbronchiolitis ist.

11. Verwendung gemäß der Ansprüche 1 - 7, wobei die therapeutisch wirksamen Peptide in Aerosolen appliziert werden.

## Claims

1. Use of a substance for synthesising a drug for the treatment of patients who suffer from idiopathic pulmonary fibrosis, hypersensitive pneumonia or diffuse panbronchiolitis, wherein said substance is a peptide or a polypeptide containing the following amino acid sequence:
Arg-Lys-Gln-Met-Ala-Val-Lys-Lys-Tyr-Leu (SEQ ID NO 4).

2. Use according to claim 1, wherein said peptide or a polypeptide further containing following amino acid sequence:
His-Ser-Asp (SEQ ID number 14); Phe-Thr-Asp (SEQ ID NO 13).

3. Use according to claim 1, wherein said peptide or a polypeptide having following amino acid sequence:
(A)ₙ-Arg-Lys-Gln-Met-Ala-Val-Lys-Lys-Tyr-Leu-(B)ₘ
wherein A and B represent independently of each other any natural occurring amino acid; and n and m are independently of each other replacing values from 0-25.

4. Use according to claim 3, wherein (A)ₙ has following sequence if n > 2:
(X)ₒ-Phe-Thr-Asp-(Y)ₚ;
wherein X and Y represent independently of each other any natural occurring amino acid; and o and p are independently of each other replacing values from 0-11.

5. Use according to claim 4, wherein (X)ₒ has following sequence if o > 2:
(X')_{q}-His-Ser-Asp-(X")ᵣ,
wherein X' and X" represent independently of each other any natural occurring amino acid; and r and q are independently of each other replacing values from 0-4.

6. Use according to claim 3, wherein the sequence of said peptide or polypeptide is selected from the following group:
(i) Arg-Lys-Gln-Met-Ala-Val-Lys-Lys-Tyr-Leu (SEQ ID NO 4)
(ii)
(iii)
(iv)
(v)
(vi)
(vii)
(viii)
(ix)
(x)
(xi)
wherein X¹ - X²² is any naturally occurring amino acid.

7. Use according to claims 1 - 6, wherein any said peptide or polypeptide is in a stabilised form.

8. Use according to claims 1 - 7, wherein said disease is idiopathic pulmonary fibrosis.

9. Use according to claims 1 - 7, wherein said disease is hypersensitive pneumonia.

10. Use according to claims 1 - 7, wherein said disease is diffuse panbronchiolitis.

11. Use according to claims 1 - 7, wherein the therapeutically effective peptides are administered as aerosols.

## Revendications

1. Utilisation d'une substance pour synthétiser un médicament pour le traitement d'un patient souffrant de fibrose pulmonaire idiopathique, pneumonie hypersensitive ou panbronchiolitis diffus, où ladite substance est un peptide ou un polypeptide contenant la suivante séquence d'acides aminés:
Arg-Lys-Gln-Met-Ala-Val-Lys-Lys-Tyr-Leu (SEQ ID numéro 4).

2. Utilisation selon la revendication 1, où ledit peptide ou polypeptide en outre contient la suivante séquence d'acides aminés :
His-Ser-Asp (SEQ ID number 14); Phe-Thr-Asp (SEQ ID numéro 13).

3. Utilisation selon la revendication 1, où ledit peptide ou polypeptide a la suivante séquence d'acides aminés :
(A)ₙ-Arg-Lys-Gln-Met-Ala-Val-Lys-Lys-Tyr-Leu-(B)ₘ;
où A, B est chacun des acides aminés naturels, A et B sont indépendants l'un de l'autre; et n, m représentent des valeurs entre 0-25; n et m sont indépendants l'un de l'autre.

4. Utilisation selon la revendication 3, où (A)ₙ a la suivante séquence quand n > 2:
(X)ₒ-Phe-Thr-Asp-(Y)ₚ,
où X, Y est chacun des acides aminés naturels, X et Y sont indépendants l'un de l'autre; et o, p représentent des valeurs entre 0-11; o et p sont indépendants l'un de l'autre.

5. Utilisation selon la revendication 4, où (X)ₒ a la suivante séquence quand o > 2 :
(X')_{q}-His-Ser-Asp-(X")ᵣ,
où X', X" est chacun des acides aminés naturels, X' et X" sont indépendants l'un de l'autre; et r, q représentent des valeurs entre 0-4; r et q sont indépendants l'un de l'autre.

6. Utilisation selon la revendication 3, où la séquence décrit auparavant est choisie du groupe suivant:
(i) Arg-Lys-Gln-Met-Ala-Val-Lys-Lys-Tyr-Leu (SEQ ID numéro 4)
(ii)
(iii)
(iv)
(v)
(vi)
(vii)
(viii)
(ix)
(x)
(xi)
où X¹ - X²² est chacun des acides aminés naturels.

7. Utilisation selon les revendications 1 - 6, où chacun des lesdits peptides ou polypeptides existe dans une forme stabilisée.

8. Utilisation selon les revendications 1 - 7, où ladite maladie est la fibrose pulmonaire idiopathique.

9. Utilisation selon les revendications 1 - 7, où ladite maladie est la pneumonie hypersensitive.

10. Utilisation selon les revendications 1 - 7, où ladite maladie est le panbronchiolitis diffus.

11. Utilisation selon les revendications 1 - 7, où les peptides thérapeutiquement effectifs sont appliqués en forme d'aérosols.
